# EUROPEAN PATENT SPECIFICATION

(11) **EP 0 607 151 B1**
(45) Date of publication and mention of the grant of the patent: **13.11.2002**
(21) Application number: 92914420.2
(22) Date of filing: 17.06.1992
(51) Int. Cl.: C07H 15/12, C12N 15/10, C12P 19/34

(54) **A METHOD OF DETECTING AND DISCRIMINATING BETWEEN NUCLEIC ACID SEQUENCES**
VERFAHREN ZUM NACHWEIS UND UNTERSCHEIDEN VON NUKLEINSÄURESEQUENZEN
PROCEDE DE DETECTION DE SEQUENCES D'ACIDE NUCLEIQUE ET DE DISCRIMINATION ENTRE CES SEQUENCES

(43) Date of publication of application: 27.07.1994
(73) Proprietor: CITY OF HOPE, Duarte California 91010-0269 (US)
(72) Inventor: WALLACE, Robert, Bruce, Greenbrae, CA 94904 (US)
(74) Representative: Marchant, James Ian
(86) International application number: US9205133
(87) International publication number: WO93025563

(56) References cited:
- EP-A- 0 416 817
- WO-A-89/10414
- WO-A-90/09455
- WO-A-90/11372
- WO-A-91/13075
- ANALYTICAL BIOCHEMISTRY, Volume 177, issued 1989, G.H. KELLER et al., "A Sensitive Nonisotopic Hybridization Assay for HIV-1 DNA", pages 27-32.
- CLINICAL CHEMISTRY, Volume 38, issued 1992, A. JALANKO et al., "Screening for Defined Cystic Fibrosis Mutations by Solid-Phase Minisequencing", pages 39-43.
- ANALYTICAL BIOCHEMISTRY, Volume 196, issued 1991, M.L. WILHELM et al., "Analysis of Mutant tRNA Gene Transcripts in Vivo in Saccharomyces Cerevisiae by Abortive Primer Extension", pages 156-160.

### SEQUENCE LISTING

(1) GENERAL INFORMATION:
   (i) APPLICANT: R. Bruce Wallace
   (ii) TITLE OF INVENTION:
      METHOD OF DETECTING AND DISCRIMINATING
      BETWEEN NUCLEIC ACID SEQUENCES
   (iii) NUMBER OF SEQUENCES: 25
   (iv) CORRESPONDENCE ADDRESS:
      (A) ADDRESSEE: City of Hope
      (B) STREET: 1500 East Duarte Road
      (C) CITY: Duarte
      (D) STATE: California
      (E) COUNTRY: United States of America
      (F) ZIP: 91010-0269
   (v) COMPUTER READABLE FORM:
      (A) MEDIUM TYPE: 3M Double Density 5 1/4" diskette
      (B) COMPUTER: Wang PC
      (C) OPERATING SYSTEM: MS-DOS (R) Version 3.30
      (D) SOFTWARE: Microsoft (R)
   (vi) CURRENT APPLICATION DATA:
      (A) APPLICATION NUMBER: To be assigned
      (B) FILING DATE: 15 June 1992
      (C) CLASSIFICATION: Unknown
   (vii) PRIOR APPLICATION DATA: NONE
   (viii) ATTORNEY/AGENT INFORMATION:
      (A) NAME: Irons, Edward S.
      (B) REGISTRATION NUMBER: 16,541
      (C) REFERENCE/DOCKET NUMBER: None
   (ix) TELECOMMUNICATION INFORMATION:
      (A) TELEPHONE: (202) 785-6938
      (B) TELEFAX: (202) 785-5351
      (C) TELEX: 440087 LM WSH
(2) INFORMATION FOR SEQ ID NO:1
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 1 5'GCAAGTTTGGCTTTTGGGGA
INFORMATION FOR SEQ ID NO: 2
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 2 5'CTGCCAAGAGGAGAAGAATG
INFORMATION FOR SEQ ID NO: 3
   (i) SEQUENCE CHARACTERISTICS:
      (A) LENGTH: 34
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 3 5'TGACGTCCATCGTCTCTGCGAATGTCTCTCCAGATTTCA
INFORMATION FOR SEQ ID NO: 4
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO:4 5'TCGCGTTGCATAAGATCCGA
INFORMATION FOR SEQ ID NO: 5
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 5 5'CTTAACGAAAGCTGCGGTCT
INFORMATION FOR SEQ ID NO: 6
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 6 5'TTCGAGCTCCAGTTAACGAG
INFORMATION FOR SEQ ID NO: 7
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 7 5'TCTCTATGTCGGAAGAGCCA
INFORMATION FOR SEQ ID NO: 8
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 8 5'CATAATGCGGTCTCGATACG
INFORMATION FOR SEQ ID NO: 9
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 9 5'ATTGCCGTCCGTAGGACTAA
INFORMATION FOR SEQ ID NO: 10
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 10 5'TCTATCACGGACTATCGGGA
INFORMATION FOR SEQ ID NO: 11
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 11 5'TTGTCACAGGCACAATGTGC
INFORMATION FOR SEQ ID NO: 12
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 12 5'CGAAGAGCCATGATGCTTCT
INFORMATION FOR SEQ ID NO: 13
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 13 5'AGACGTCGTCACGATTCTGA
INFORMATION FOR SEQ ID NO: 14
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 14 5'ACACGTGCGCCTGGAATTAT
INFORMATION FOR SEQ ID NO: 15
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
      (K) RELEVANT RESIDUES IN SEQ ID NO: 15:
   (xi) SEQUENCE DESCRIPTION: SEQ ID NO: 15 5'CAGGTGCCATATAATGGTCC
INFORMATION FOR SEQ ID NO: 16
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 16 5'TGGCCATACCAGACTTTAGG
INFORMATION FOR SEQ ID NO: 17
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 17 5'ATGGGCTCCTGCGTAAATCA
INFORMATION FOR SEQ ID NO: 18
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 18 5'AGTGCGCTCTCTTGAGCAAA
INFORMATION FOR SEQ ID NO: 19
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 19 5'ACTGTTACCGGTAACTGACG
INFORMATION FOR SEQ ID NO: 20
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 20 5'GATATACCATTCCAGGCGTG
INFORMATION FOR SEQ ID NO: 21
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 21 5'TCCGGCTCATGGTAGAATAC
INFORMATION FOR SEQ ID NO: 22
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 22 5'CTTACAATATCCGGCGTGGA
INFORMATION FOR SEQ ID NO: 23
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 23 5'GGGTATTCTCGACCAATCAG
INFORMATION FOR SEQ ID NO: 24
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 24 5'AAATTTTTTTGGCCCCCC
INFORMATION FOR SEQ ID NO: 25
   (i) SEQUENCE CHARACTERISTICS:
      (A) LENGTH: 20
      (B) TYPE: Nucleotide
      (C) STRANDEDNESS: Single
      (D) TOPOLOGY: Linear
   (ii) MOLECULE TYPE: DNA
   (vii) IMMEDIATE SOURCE: Synthetically prepared
   (viii) POSITION IN GENOME: None
   (ix) FEATURE: None
   (x) PUBLICATION INFORMATION: None
   (xi) SEQUENCE DESCRIPTION: SEQ ID NO: 25

5'GGGGGGGCCCAAAAAATTTT).

## Description

### FIELD OF THE INVENTION

The present invention relates to the use of a solid support for detecting nucleic acid sequences and a method of detecting nucleic acid sequences. More specifically the invention relates to a method for determining if a particular DNA or RNA sequence is present in a sample. The invention also relates to discriminating between sequences which differ from each other by as little as a single nucleotide. The DNA or RNA may be single or double stranded, and may be a single species or a component of a mixture of nucleic acids. The method of the invention utilizes a novel primer design. The sequence of the primer is composed of two portions, the 3' portion is a primer specific for the target nucleic acid sequence and the 5' portion is complementary to a preselected nucleic acid sequence. Extension of the 3' portion of the primer with labeled deoxynucleosides triphosphate yields a labeled extension product if, but only if, the template includes the target sequence. The labeled extension product is detected by hybridization of the 5' portion to the preselected sequence.

### BACKGROUND OF THE INVENTION

The genome of an organism is unique. Not only do the genomes of different species differ, but the genomes of different individuals within a species differ (with the exception of identical twins or clones). These differences provide individual and species specific characteristics which can be used for identification by nucleic acid biochemical techniques such as hybridization and polymerase mediated reactions, both dependent for their specificity on precise base pairing.

The goal of nucleic acid based diagnostics is the detection of specific nucleic acid sequences. This goal often requires the detection of a specific sequence in the presence of other sequences. In certain cases it is necessary to discriminate between closely related sequences, even sequences which differ by only a single nucleotide. Prior art methods for doing so are described in various publications. For example, the use of allele-specific oligonucleotide (ASO) hybridization probes for the detection of specific nucleic acid sequences has been described (Wu et al., DNA 8:135-142 (1989); Thein, et al., Br. J. Haematol. 70:225-231 (1988); Connor, et al., Proc. Natl. Acad. Sci. USA 80:278-282 (1983); Studencki, et al., Am. J. Hum. Genet. 37:42-51 (1985); Pirastu, et al., N.Engl.J.Med. 309:284-287 (1983); Orkin, et al., J.Clin.Invest. 71:775-779 (1983); Thein and Wallace, The use of synthetic oligonucleotides as specific hybridization probes in the diagnosis of genetic disorders. In Human genetic diseases: A practical approach. K.E. Davies, ed. (Oxford; IRL Press), pp. 33-50 (1986)). This approach allows the discrimination between nucleic acids which differ by as little as a single nucleotide (e.g., alleles). Individual hybridization reactions are required for each allele to be detected. Erlich, et al., Eur.J.Immunogenet. 18:33-55 (1991) and Zhang, et al., Nucleic Acids Res. 19:3929-3933 (1991) have recently described the use of immobilized ASO probes. In this method, a set of ASO probes is immobilized on a membrane and hybridized with labeled polymerase chain reaction (PCR) products. Under appropriate conditions hybridization is allele specific. Each hybridization can analyze only a single amplification reaction. The present invention allows for the detection of specific sequences in a sample. Because the template specific step and the detection step are each controlled by specific but independent base pairing requirements, the overall process allows detection of multiple templates and multiple samples simultaneously.

The concept of in vitro DNA amplification was first proposed by Khorana and coworkers in 1971 (Kleppe, et al., J.Mol.Biol. 56:341-361 (1971)). Realizing that total chemical synthesis of a gene would result in a finite amount of product, a procedure for in vitro replication was proposed. Their procedure was based on extensive studies of the repair replication reaction, the in vitro replication of a DNA template using a complementary primer (Kleppe, supra). Their proposal was as follows: "The DNA duplex would be denatured to form single strands. This denaturation step would be carried out in the presence of a sufficiently large excess of the two appropriate primers. Upon cooling, one would hope to obtain two structures, each containing the full length of the template strand appropriately complexed with the primer. DNA polymerase will be added to complete the process of repair replication. Two molecules of the original duplex should result. The whole cycle could be repeated, there being added every time a fresh dose of the enzyme." More recently, this in vitro amplification process has been further developed into the polymerase chain reaction (Mullis, et al., Cold Spring Harbor Symp. Quant. Biol. 51:263-273 (1986); Saiki, et al., Science 230:1350-1354 (1985); U.S. Patent No. 4,683,202). Although template amplification improves detection of a particular sequence because a larger amount of template is available for analysis, post amplification steps are often required to detect specific sequences within the amplified product. For example, ASO hybridization has been combined with PCR amplification for the specific detection of various disease alleles (Impraim, et al., Biochem. Biophys. Res. Commun. 142:710-716 (1987); Saiki, et al., Nature 324:163-166 (1986); Farr, et al., Proc.Natl. Acad.Sci. USA 85:1629-1633 (1988); Saiki, et al., N.Engl.J.Med. 319:537-541 (1988); Chehab, et al., Nature 329:293-294 (1987)).

WO90/11372 discloses a method in which a test sample of DNA is treated with an oligonucleotide primer, in which the primer is capable of pairing with a first portion of the DNA adjacent to the test nucleotide. The primer of this reference includes a nucleotide at the position opposite the test nucleotide when the primer and the DNA are paired. The primer-DNA pair is then treated with conditions which allow the construction of an extension product complimentary to a second portion of the DNA if there is a base-pair match at the test nucleotide position, but not if there is a mismatch. The presence or absence of the extension product is then determined.

The present invention provides an alternative for the analysis of PCR amplification products to determine the presence or absence of specific sequences.

### SUMMARY OF THE INVENTION

This invention provides a method for determining the presence or absence of a target nucleic acid sequence present in a sample and for discriminating between any two nucleic acid sequences even if such sequences differ only by a single nucleotide. The nucleic sequences may be single or double stranded DNA or RNA. The target sequence may be relatively pure species or a component of a nucleic acid mixture. It may be produced by an in vitro amplification such as a PCR or ligation amplification reaction or by a plurality of cycles of primer extension.

The method of the invention entails extension of a novel two component primer on templates which may or may not include a target nucleic acid sequence.

According to the invention there is provided the use of a solid support for capturing a plurality of nucleic acids for detecting the presence or absence of an allele specific nucleotide in a target nucleic acid sequence, wherein each of said nucleic acids has a 3' portion complementary to a target sequence immediately adjacent on allele specific nucleotide and a 5' portion, wherein said solid support comprises a plurality of immobilized and preselected nucleic acid sequences, wherein each of said immobilized preselected nucleic acid sequences is capable of hybridising to the complementary 5' portion of the captured nucleic acid under the same hybridisation conditions for all said immobilized preselected nucleic acid sequences.

According to the invention there is further provided a method for detecting the presence or absence of an allele specific nucleotide in a target nucleic acid sequence in a nucleic acid sample, said method comprising
(a) combining under hybridising conditions to form a duplex
   (i) a nucleic acid sample which may include a target nucleic acid sequence,
   (ii) an allele specific primer having a 3' portion and a 5' portion wherein said 3' portion is complementary to a target sequence immediately adjacent on allele specific nucleotide and wherein said 5' portion is complementary to a preselected nucleic acid which is different from said target sequence and wherein said preselected nucleic acid sequence is immobilized at a preselected location in an array of immobilized and preselected nucleic acid sequences and solid support,
(b) adding to the product of step (i) and DNA polymerase and deoxynucleoside triphosphates, one of which is an allele specific nucleotide containing a label, under hybridisation conditions to provide a labelled extension product of said primer which includes said target sequence and said allele specific nucleotide if present;
(c) screening allele specific labelled primer extension product of (b) by combining the 5' portion with the array of immobilized and preselected nucleic acid sequences on a solid support whereby the label identifies the allele specific nucleotide and the location of the immobilized preselected nucleic acid sequence is associated with the locus of the target nucleic acid sequence in the nucleic acid sample.
The 3' portion of the primer is complementary to a portion of the template adjacent the target sequence. The 5' portion of the primer is complementary to a different preselected nucleic acid sequence. Extension of the 3' portion of the primer with a labeled deoxynucleoside triphosphates yields a labeled extension product if, but only if, the template includes the target sequence. The presence of such a labeled primer extension product is detected by hybridization of the 5' portion to the preselected sequence. The preselected sequence is preferably immobilized on a solid support. A plurality of preselected sequences immobilized on a solid support to provide an array for concurrent screening of a plurality of labeled primer extension products is provided.

One practical embodiment of the invention relates to methods for diagnosing diseases such as sickle cell anemia or thalassemia caused by a defective allele. Kits for performing such a diagnosis are provided.

### DESCRIPTION OF THE FIGURES

Figure 1 depicts a two component primer useful in this invention.
Figure 2 depicts one embodiment of the invention.
Figure 3 depicts a solid support having a plurality of preselected sequences immobilized in a particular array on a grid.
Figure 4 depicts the application of the present invention for the detection of alleles of the human TYR locus.
Figure 5 depicts the genotypic analysis of ten individuals for alleles at the TYR locus using the invention.

### DEFINITIONS

As used herein, the following words have the meaning set forth:
"Oligonucleotide" refers to a nucleic acid sequence composed of two or more nucleotides. It can be derived from natural sources but is often synthesized chemically. It can be of any length. It is generally used as a primer, a probe or a component of ligation reaction.
"Primer" refers to an oligonucleotide which is used to initiate nucleic acid synthesis by a template dependent polymerase such as a DNA polymerase. The primer is complementary to a portion of a template nucleic acid.
"Primer extension" refers to the process of elongation of a primer on a nucleic acid template. Using appropriate buffers, pH, salts and nucleoside triphosphates, a template dependent polymerase such as a DNA polymerase incorporates a nucleotide complementary to the template strand on the 3' end of a primer which is annealed to a template. The polymerase will thus synthesize a faithful complementary copy of the template. If only a single nucleoside triphosphate is present in a primer extension reaction, then that nucleotide will be incorporated in the primer extension product only if the base of the nucleoside triphosphate is complementary to the base of the template immediately adjacent to the 3' end of the primer.
"Allele" refers to one of two or more alternative forms of a gene occupying corresponding sites (loci) on homologous chromosomes. The DNA sequence of alleles of a locus differ from each other by at least one nucleotide.
"Target" refers to the nucleic acid sequence to be detected or discriminated.
"Sample" refers to a nucleic acid which may or may not contain the target. The sample nucleic acid may be DNA or RNA, single or double stranded and present in a relatively pure form in the sample or one component of a mixture in the sample. In the case of RNA it is often useful to convert the RNA into DNA using a reverse transcription step. The DNA product can then be analyzed directly or subjected to an amplification step prior to analysis by the present invention.
"Allele specific primer extension (ASPE)" refers to a method in which an oligonucleotide primer is annealed to a DNA template 3' with respect to a nucleotide indicative of the presence or absence of a target allelic variation. The primer is then extended in the presence of labelled dNTP in which the N is complementary to the nucleotide indicative of the presence or absence of the target allelic variation in the template.

### DETAILED DESCRIPTION OF THE INVENTION

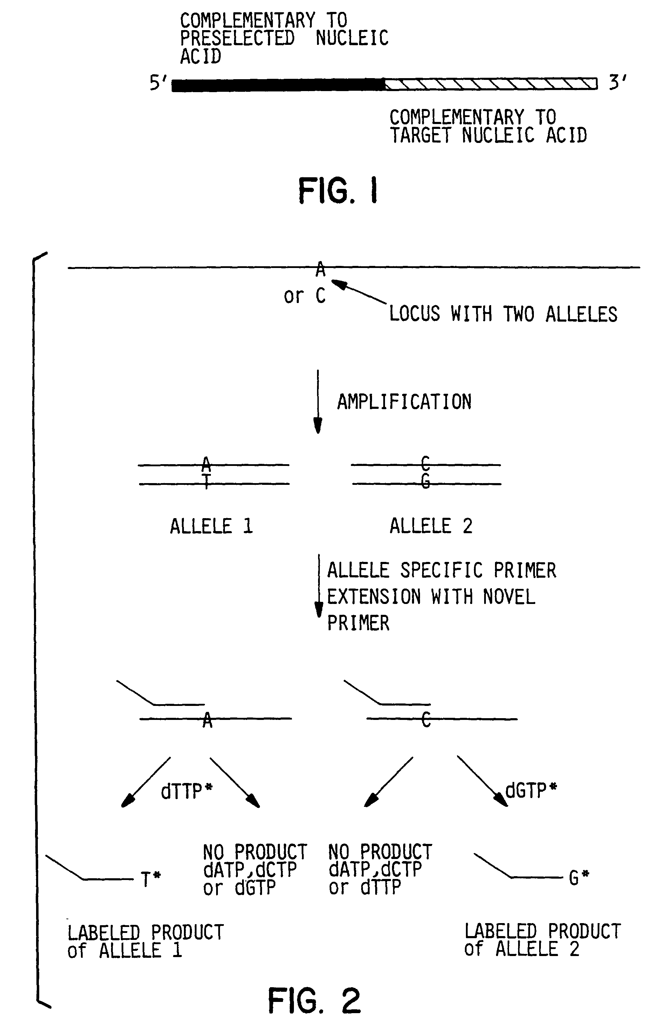

The invention is described by reference to the Figures. As shown by Figure 1, the 3' portion of the primer is complementary to a sequence adjacent a target sequence which may or may not be present in a sample. The 5' portion of the primer is complementary to a known or preselected sequence preferably immobilized on a solid support and arranged in a particular pattern. The two components of the primer may have any desired number of nucleotides. The number of nucleotides in each portion of the primer may be the same or different. Preferably each portion of the primer contains from about 10 to about 100 nucleotides. The word "about" indicates a variance e.g., of plus or minus ten nucleotides.

Figure 2 illustrates the principle of the invention as applied to identify and discriminate between two allelic nucleic acid sequences which may be present in a sample and which differ by a single nucleotide. As illustrated, a sample may contain either "A-T" or "C-G" alleles.

The primer is hybridized to the alleles immediately adjacent the variant nucleotides responsible for the allelism. ASPE reactions are performed with each of four labeled deoxynucleoside triphosphates independently. Only one of the primer extension reactions will label the primer for each of the different alleles.

As specifically depicted by Figure 2, the amplification product is subjected to hybridization with a primer which includes a 3' portion complementary to the "A-T" allele which is then subjected to extension with labeled dNTP. Extension will occur only with dTTP if the A-T allele is present in the sample. No extension occurs with ATP, dCTP or dGTP.

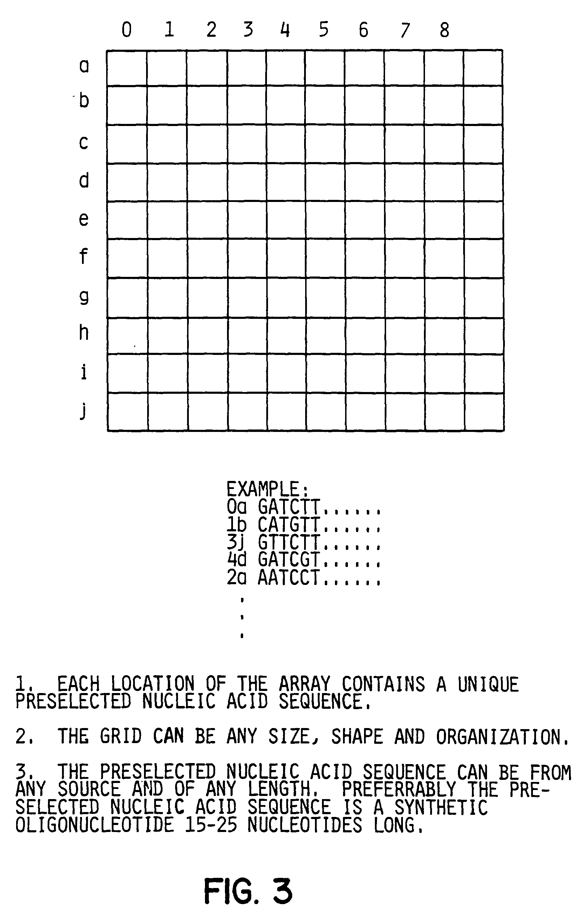

The labeled primer extension product is screened by hybridization of the 5' portion of the labeled primer with the solid support depicted by Figure 3. Each location on the array of sequences contains a different preselected oligonucleotide. The solid support may be of any size with any desired number of locations. The preselected oligonucleotides immobilized at each of the grid locations are preferably at least 10-100, preferably 15 to 25 nucleotides in length. Hybridization preferably, but not necessarily, occurs under substantially the same conditions at each location on the array.

The present invention is suitable for the detection of any nucleic acid. For example, if a sample were suspected of containing a nucleic acid specific for a pathogen, the sample could be analyzed by the present invention. A novel primer would be synthesized with a 3' portion specific for the pathogen genome and a 5' portion complementary to a preselected sequence. The sample would be either analyzed directly or after amplification by PCR.

The present invention is also suitable for discriminating between individuals on the basis of genetic differences. For example, the determination of which alleles are preset at 20 different dimorphic, genetically unlinked loci would provide a powerful method useful in forensic science to discriminate between different individuals. The present invention is useful, e.g., in transplantation medicines, to determine which HLA alleles are present in a DNA sample of an individual.

### EXEMPLIFICATION OF THE INVENTION

### EXAMPLE I

### Experimental Design

The first step, not shown, involves the amplification of the target sequence with a primer set (TYR 1 and TYR 2) specific for the TYR locus. After the amplification, the template is prepared for the next step by eliminating the remaining dNTPs.

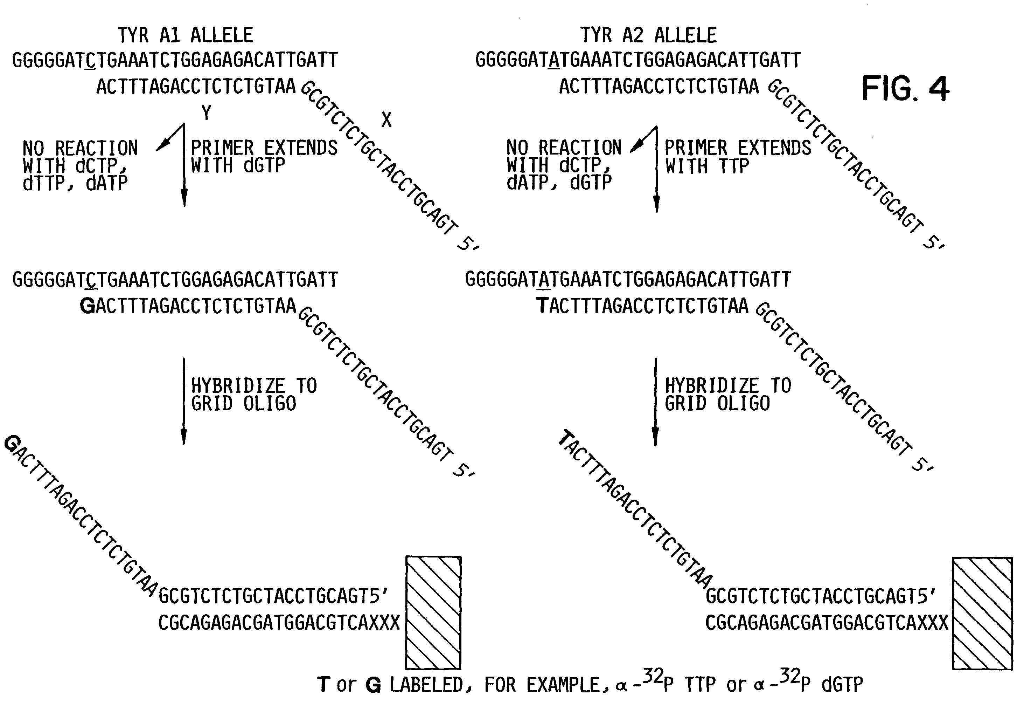

Figure 4 shows an overview of the procedure called ASPE-capture, applied in this example to the detection of the amplified dimorphic sequence present at the TYR locus produced by the first step.

The method consists of three steps. This procedure is locus and allele specific in that it is designed both to specifically detect the presence of the amplification product and to determine the target identifying polymorphic nucleotide of the analyzed locus. Figure 4 depicts two separate primer extension reactions which have as common ingredients the DNA template, the ASPE primer, and the Ampli-Taq polymerase. The two reactions differ in that they contain different nucleoside triphosphates: allele A1 is detected by including α-³²P dGTP and allele A2 by including α-³²P TTP. The ASPE primer is a 40 nucleotide long oligonucleotide which includes two different portions (X and Y) each having a different role. Sequence Y is identical to the sense sequence of the TYR gene with its 3' nucleotide immediately flanking the polymorphic base. This portion of the ASPE primer participates in the primer extension reaction. Y sequence is extended by the Ampli-Taq DNA polymerase whenever an α-³²P labeled nucleotide complementary to the polymorphic base is present in the reaction. For a DNA template from individuals homozygous for the TYR-A1 allele, the ASPE primer will be only extended by α-³²P labeled dGTP. α-³²P labeled TTP will be added to the ASPE primer when a DNA template from individuals homozygous for the TYR-A2 allele is analyzed. Both labeled nucleoside triphosphates will be added to the ASPE primer for DNA templates from heterozygous individuals. It should be noted that if no template is present, no labeling of the ASPE primer will happen. Sequence X is complementary to a grid oligonucleotide, thus permitting capture of the primer extension product on the grid.

This invention is not limited to the described steps. For example, the labeling of the primer extension product could be accomplished using fluorescently labeled deoxynucleoside triphosphates or dideoxynucleoside triphosphates. The capture could be accomplished by hybridization in solution to an oligonucleotide which can later be immobilized (for example by utilizing biotin-avidin, hapten-antibody reactions).

### EXAMPLE II

### Synthesis of Oligonucleotides

### Chemical synthesis of oligonucleotide primers

Oligonucleotides were synthesized on a Cruachem PS 250 DNA synthesizer. Oligonucleotides for amplifying the target sequence (TYR 1 and TYR 2) were purified by HPLC. The oligonucleotide used in the allele specific primer extension reactions (ASPE primer) was purified by HPLC followed by a 20% polyacrylamide gel/7M urea. The sequences of the synthetic oligonucleotides utilized are presented in Table 1.

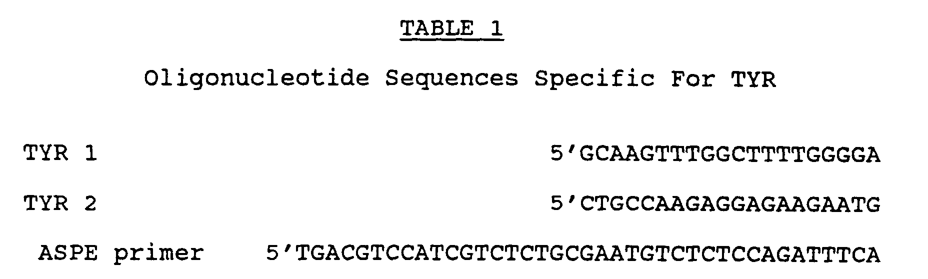

### Chemical synthesis of grid oligonucleotide

The grid oligonucleotide for attachment to the nylon membrane was prepared as follows: 3'Amine-ON CPC columns (0.2 µmole) were purchased from Cruachem and were used on the Cruachem PS 250 DNA synthesizer. Three 1,3 propanediol nucleoside substitutes were coupled directly to the column prior to coupling the grid oligonucleotide sequence. The protected 1,3 propanediol phosphoramidite was prepared essentially as described by Seela and Kaiser, Nucleic Acids Res. 15:3113-3129 (1987). This phosphoramidite was placed in the X position of the synthesizer. For example to synthesize the grid oligonucleotide for the detection of TYR extension products, the synthesizer was programmed to synthesize 5'CGCAGAGACGATGGACGTCAXXX. The oligonucleotide is deprotected as usual and the oligonucleotide recovered by ethanol precipitation. The oligonucleotide contains a 3'NH₂ group.

### Preparation of the filters containing grid oligonucleotides

The membranes containing the grid oligonucleotides were prepared according to Zhang, et al., Nucleic Acids Res. 19:3929-3933 (1991). Briefly, Biodyne C membranes (Pall Biosupport, NJ) were rinsed with 0.1N HC1 and then treated for 15 minutes with 20% EDC (1-Ethyl-3-[dimethylamion propyl] carbodiimide hydrochloride) (w/v) in deionized water. After the activation process, the membranes were immediately set in a 96 well Bio-dot apparatus (Bio-Rad, Richmond, CA). The grid oligonucleotide was mixed with 0.5 M sodium bicarbonate buffer pH 8.4 and applied to the membranes for 15 minutes. The membranes were rinsed with TBS/0.1% Tween-20 (registered trademark) (Tris buffered saline) and then treated with 0.1 N NaOH for 10 minutes. Finally, filters were rinsed with deionized water.

### EXAMPLE III

### Allele Specific Detection Reactions

### Isolation of genomic DNA:

Blood samples were collected from ten unrelated individuals. High molecular weight DNA was prepared according to a modified procedure using Triton X-100 (registered trademark) followed by Proteinase K and RNAse treatment (Bell, et al., Proc. Natl. Acad. Sci. USA 78: 5759-5763 (1981)).

### In vitro amplification of DNA template:

DNA (50ng) from ten random individuals was amplified with a primer set derived from exon I of the human tyrosinase gene (TYR) (GenBank Accession M27160; Locus HUMTYRA) in a 50 µl reaction volume containing 50 mM KCl, 10 mM Tris-HCl (pH 8.3), 1.5 mM MgCl₂, 0.01% (w/v) gelatin, 10 pmol of each primer, 0.2 mM each dATP, dCTP, dGTP, TTP, and 2.5 units of Ampli-taq polymerase (Perkin Elmer Cetus). The reaction was performed in a thermal cycler (Perkin Elmer). The reaction mixture was denatured at 94°C for 3 minutes, then the cycle was continued by annealing the primers at 65°C for 1 minute and extending the primers at 72°C for 1 minute. After extension, the samples were denatured at 94°C for 30 seconds. The annealing, extension, and denaturation cycle was repeated 40 times. After the last cycle, the samples were incubated at 65°C for 1 minute, at 72°C for 6 minutes and finally at 4°C until analyzed.

### Preparation of DNA template for primer extension reactions:

In order to control the quality of the PCR products, 5 µl were loaded in a 1.5% agarose gel. The remaining 45 µl were ethanol precipitated twice using 2.5 M ammonium acetate and 10 µg of glycogen. The pellet was resuspended in H₂O (100 µl).

### Allele-specific primers extension reactions:

Each allele-specific primer extension reaction (ASPE) were performed in a 10 µl volume containing 1 µl of PCR template, 1 µl of 10X PCR buffer (1X=10mM Tris-HCl pH 8.3, 50 mM KCl, 1.5 mM MgC12), 0.01% (w/v) gelatin, 0.25 µM of ASPE primer, 1 unit of Ampli-taq polymerase, and 0.5 µl of the appropriate α-³²P-labeled nucleotide (10µCi/µl, 3000 Ci/mmol). To analyze the dimorphism present at locus TYR, each sample was subjected to two separate primer extension reactions using α-³²P labeled dGTP and TTP respectively. Mixtures were denatured at 94°C for 3 minutes and then subjected to 10 cycles consisting of 1 second annealing at 55°C and 30 seconds denaturation at 94°C. After the reaction, samples were ethanol precipitated and used for hybridization.

### Hybridization and post hybridization washes:

All the hybridizations were performed overnight at 55°C in 2 ml microcentrifuge tubes using hybridization incubator (Robbins Scientific, model 310). The hybridizations were done in a 225 µl volume (Hybridization solution: 5x SSPE [1x SSPE=10 mM sodium phosphate pH 7.0, 0.18 M NaCl and 1 mM EDTA], 1% SDS, 0.5% (w/v) dehydrated powdered skim milk (Carnation, Los Angeles, CA), 10 µg/ml homomix RNA, and the product of the primer extension reaction). After hybridization, the filters were washed with 6X SSC (1X SSC = 0.15 M NaCl, 0.015 M sodium citrate) at room temperature for 15 minutes. Autoradiography of membranes was done using Kodak X-AR5 film and exposing for 15-30 minutes at room temperature. Subseguently, the amount of cpm corresponding to the labelled ASPE bound to the filter was measured by Ambis-scan (Ambis Corp., San Diego, CA).

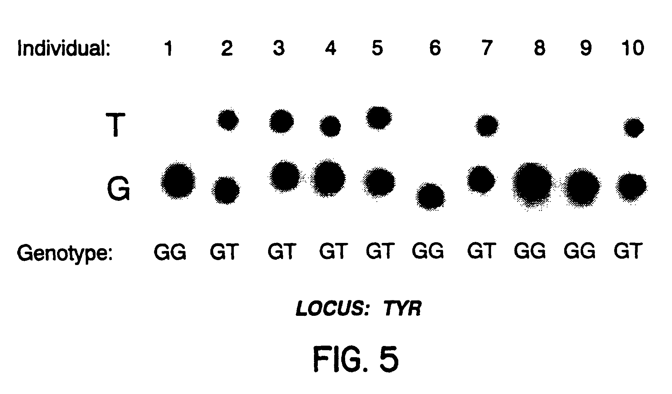

The results of hybridization are shown in Figure 5. Note that of the ten individuals analyzed, six are heterozygous (2, 3, 4, 5, 6 and 10) and four are homozygous for the TYR-A1 allele.

### EXAMPLE IV

### Grid Oligonucleotides

### General approach for the design of grid design of grid oligonucleotide sequences

Grid oligonucleotides were designed to be 20 nucleotides in length and to have a base composition of 50% G+C. This design allows hybridization reactions to be carried out with a single hybridization temperature for all sequences. The first 20 grid oligonucleotide sequences were generated by starting with the sequence 5'GGGGGCCCCCTTTTTAAAAA (25% of each of the four bases). This sequence was then randomized by the RANDOMIZATION option (group length 1, randomize all) within the program GENED in the Intellegenetic Suite software for molecular biology (Intelligenetics, Mountain View, CA) to give the first sequence in Table 2. Each of the next 19 sequences were generated by randomizing the previous sequence in the list.

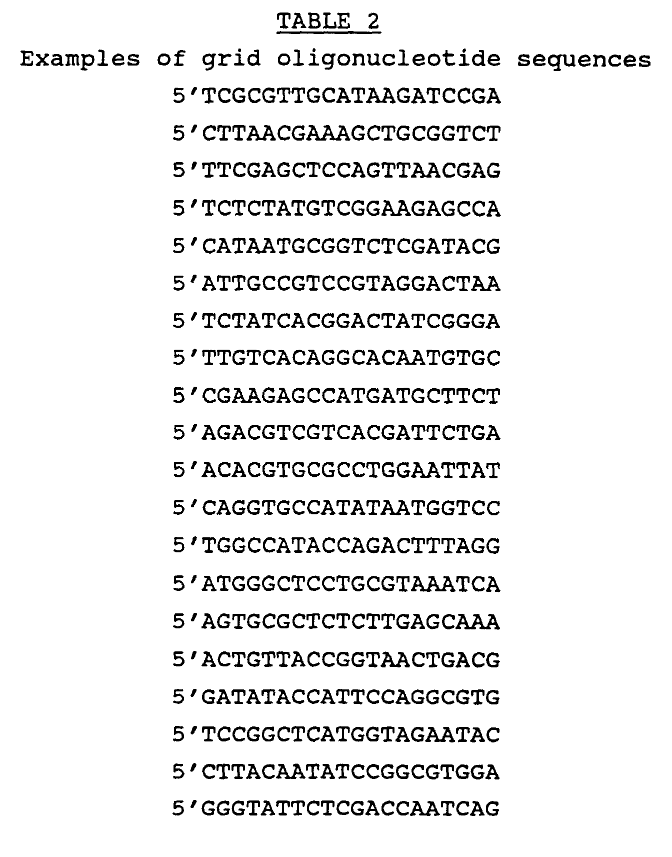

Similarly, additional grid oligonucleotides can be generated starting with other sequences which are 50% G-C (e.g., 5'AAATTTTTTTGGGCCCCCCC, 5'GGGGGGGCCCAAAAAATTTT).

### EXAMPLE V

### Removal of Deoxynucleoside Triphosphates

An important step in the practice of this invention is the removal of the deoxynucleoside triphosphates (dNTPs), if present, from the sample to be subjected to the primer extension reaction. This can be accomplished by several methods including ethanol precipitation of the template DNA (as described in Example III), by destruction of the dNTPs with an enzyme or by chromatography on a column which can separate polymerized nucleic acids from dNTPs.

In the latter approach, a column of DE52 was prepared. DE52 (Whatman) was suspended in a buffer containing 10 mM Tris-HCl, pH 7.5 (TE) such that the ratio of DE52 to buffer was 1:2. The DE52 suspension (0.2 ml) was poured into a 1 ml micropipette tip (e.g., the blue tip for Gilson P-1000 Pipetman™) which had been plugged with glass wool and washed with 1 ml of TE. The sample (e.g., the product of a polymerase chain reaction) was diluted to 0.1 ml with TE and applied to the column under slight air pressure. The column was washed with 1 ml of TE. The dNTPs were eluted with 4 X 0.5 ml of 0.2 M NaCl. Finally, the sample was recovered, essentially free of dNTPs by 0.2 ml 1M NaCl. The sample could then be used directly in a primer extension reaction.

## Claims

1. The use of a solid support for capturing a plurality of nucleic acids for detecting the presence or absence of an allele specific nucleotide in a target nucleic acid sequence, wherein each of said nucleic acids has a 3' portion complementary to a target sequence immediately adjacent on allele specific nucleotide and a 5' portion, wherein said solid support comprises a plurality of immobilized and preselected nucleic acid sequences, wherein each of said immobilized preselected nucleic acid sequences is capable of hybridising to the complementary 5' portion of the captured nucleic acid under the same hybridisation conditions for all said immobilized preselected nucleic acid sequences.

2. The use of a claim 1, wherein the location of immobilized preselected nucleic acid sequences is associated with a locus of the target sequence.

3. The use of claim 1, wherein each of the preselected nucleic acid sequences is an oligonucleotide immobilized at a preselected location in an array of immobilized oligonucleotides.

4. A method for detecting the presence or absence of an allele specific nucleotide in a target nucleic acid sequence in a nucleic acid sample, said method comprising
(a) combining under hybridising conditions to form a duplex
(i) a nucleic acid sample which may include a target nucleic acid sequence,
(ii) an allele specific primer having a 3' portion and a 5' portion wherein said 3' portion is complementary to a target sequence immediately adjacent on allele specific nucleotide and wherein said 5' portion is complementary to a preselected nucleic acid which is different from said target sequence and wherein said preselected nucleic acid sequence is immobilized at a preselected location in an array of immobilized and preselected nucleic acid sequences and solid support,
(b) adding to the product of step (i) and DNA polymerase and deoxynucleoside triphosphates, one of which is an allele specific nucleotide containing a label, under hybridization conditions to provide a labelled extension product of said primer which includes said target sequence and said allele specific nucleotide if present;
(c) screening allele specific labelled primer extension product of (b) by combining the 5' portion with the array of immobilized and preselected nucleic acid sequences on a solid support whereby the label identifies the allele specific nucleotide and the location of the immobilized preselected nucleic acid sequence is associated with the locus of the target nucleic acid sequence in the nucleic acid sample

5. The method of claim 4, wherein the preselected nucleic acid sequence is an oligonucleotide immobilized at a preselected location in an array of immobilized oligonucleotides on a solid support.

6. The method of claim 4, wherein said nucleic acid sample may contain a plurality of target nucleic acid sequences and wherein said nucleic acid sample is subjected to polymerase chain reaction amplification with at least one pair of oligonucleotide primers for two or more of said target nucleic acid sequences.

7. The method of claim 6, wherein said target nucleic acid sequences are alleles of one another, the 3' portion of said primer is positioned immediately adjacent to the variant nucleotide responsible for the allelism, and said primer extension reactions are independently performed with each of the four deoxynucleoside triphospates such that only one primer extension reaction will occur for each of said alleles, said deoxynucleoside triphosphate being labelled whereby a single labelled primer extension product is produced for each of said alleles.

8. A method as claimed in claim 4 wherein the 5' portion is complementary to a preselected and random generated nucleic acid sequence.

9. A method as claimed in claim 4 or claim 8 wherein the lengths, base composition and percentage of each base in each of said immobilized preselected nucleic acid sequence are substantially the same to permit hybridisation between said 5' portion and said preselected nucleic acid sequence to be carried out at the same temperature for all said immobilized preselected nucleic acid sequences.

## Patentansprüche

1. Verwendung eines festen Trägers zum Fangen einer Vielzahl von Nukleinsäuren zum Nachweis der Anwesenheit oder Abwesenheit eines Allel-spezifischen Nukleotids in einer Nukleinsäurezielsequenz, worin jede der Nukleinsäuren einen 3'-Bereich hat, der komplementär zu einer Zielsequenz ist, die unmittelbar an das Allel-spezifische Nukleotid angrenzt, und einen 5'-Bereich, worin der feste Träger eine Vielzahl von immobilisierten und vorselektierten Nukleinsäuresequenzen umfasst, worin jede der immobilisierten vorselektierten Nukleinsäuresequenzen in der Lage ist, unter denselben Hybridisierungsbedingungen für alle immobilisierten, vorselektierten Nukleinsäuren an den komplementären 5'-Bereich der gefangenen Nukleinsäure zu binden.

2. Verwendung nach Anspruch 1, worin der Ort der immobilisierten vorselektierten Nukleinsäuren mit einem Locus der Zielsequenz assoziiert ist.

3. Verwendung nach Anspruch 1, worin jede der vorselektierten Nukleinsäuresequenzen ein immobiliertes Oligonukleotid an einem vorselektierten Ort in einer Anordnung immobilisierter Oligonukleotide ist.

4. Verfahren zum Nachweis der Anwesenheit oder Abwesenheit einer Allel-spezifischen Nukleinsäure in einer Nukleinsäurezielsequenz in einer Nukleinsäureprobe, wobei das Verfahren
(a) das Kombinieren zum Ausbilden einer Duplex unter Hybridisierungsbedingungen
(i) einer Nukleinsäureprobe, die eine Nukleinsäurezielsequenz enthalten kann,
(ii) eines Allel-spezifischen Primers, der einen 3'-Bereich und einen 5'-Bereich hat, worin der 3'-Bereich komplementär zu einer auf dem Allel-spezifischen Nukleotid unmittelbar angrenzenden Zielsequenz ist und worin der 5'-Bereich komplementär zu einer vorselektierten Nukleinsäure ist, die sich von der Zielsequenz unterscheidet und worin die vorselektierte Nukleinsäuresequenz an einem vorselektierten Ort in einer Anordnung von immobilisierten und vorselektierten Nukleinsäuresequenzen und festem Träger immobiliert ist,
(b) die Zugabe zu dem Produkt aus Schritt (i) und DNA-Polymerase und Desoxynukleosidtriphosphate, von denen eins ein Allel-spezifisches Nukleotid ist, das einen Marker trägt, unter Hybridisierungsbedingungen, um ein markiertes Primerextensionsprodukt zu erhalten, das die Zielsequenz und, falls vorhanden, das Allel-spezifische Nukleotid enthält,
(c) Screening der Allel-spezifischen markierten Primerextensionsprodukte aus (b) durch Kombinieren des 5'-Bereichs mit der Anordnung immobilisierter und vorselektierter Nukleinsäuresequenzen auf einem festen Träger, wobei der Marker die Allel-spezifischen Nukleotide erkennt und der Platz der immobilisierten, vorselektierten Nukleinsäuresequenzen mit dem Locus der Nukleinsäurezielsequenz in der Nukleinsäureprobe assoziiert ist, umfasst.

5. Verfahren nach Anspruch 4, worin die vorselektierte Nukleinsäuresequenz ein am vorselektierten Ort auf einem festen Träger in einer Anordnung immobilisierter Oligonukleotide immobilisiertes Oligonukleotid ist.

6. Verfahren nach Anspruch 4, worin die Nukleinsäureprobe eine Vielzahl von Nukleinsäurezielsequenzen enthalten kann und worin die Nukleinsäureprobe einer Polymerasekettenreaktion-Amplifizierung mit mindestens einem Paar Oligonukleotidprimem von zwei oder mehreren der Nukleinsäurezielsequenzen unterworfen wird.

7. Verfahren nach Anspruch 6, worin die Nukleinsäurezielsequenzen Allele voneinander sind, der 3'-Bereich des Primers unmittelbar benachbart zu den verschiedenen Nukleotiden, die für den Allelismus verantwortlich sind, angeordnet ist und die Primerextensionsreaktionen unabhängig mit jedem der vier Desoxynukleosidtriphosphate durchgeführt werden, so dass nur eine Primerextensionsreaktion für jedes der Allele erfolgt, wobei das Desoxynukleosidtriphoshate markiert wird, wodurch ein einziges markiertes Primerextensionsprodukt für jedes der Allele hergestellt wird.

8. Verfahren nach Anspruch 4, worin der 5'-Bereich zu einer vorselektierten und zufällig gebildeten Nukleinsäuresequenz komplementär ist.

9. Verfahren nach Anspruch 4 oder Anspruch 8, worin die Längen, die Basenzusammensetzungen und der Prozentgehalt jeder Base in jeder immobilisierten, vorselektierten Nukleinsäuresequenz im wesentlichen dieselben sind, um die Durchführung der Hybridisierung zwischen dem 5'-Bereich und der vorselektierten Nukleinsäuresequenz für alle immobilisierten, vorselektierten Nukleinsäuresequenzen bei derselben Temperatur zu ermöglichen.

## Revendications

1. Utilisation d'un support solide pour capturer une pluralité d'acides nucléiques, pour déterminer la présence ou l'absence d'un nucléotide spécifique d'allèle dans une séquence d'acides nucléiques cibles, où chacun desdits acides nucléiques a une portion 3' complémentaire d'une séquence cible immédiatement adjacente aux nucléotides spécifiques d'allèle et une portion 5', où ledit support solide comprend une pluralité de séquences d'acides nucléiques immobilisées et présélectionnées, où chacune desdites séquences d'acides nucléiques présélectionnées immobilisées est capable de s'hybrider à la portion 5' complémentaire de l'acide nucléique capturé dans les mêmes conditions d'hybridation pour toutes lesdites séquences d'acides nucléiques présélectionnées immobilisées.

2. Utilisation selon la revendication 1, pour laquelle l'emplacement des séquences d'acides nucléiques présélectionnées immobilisées est associé à un locus de la séquence cible.

3. Utilisation selon la revendication 1, pour laquelle chacune des séquences d'acides nucléiques présélectionnées est un oligonucléotide immobilisé sur un emplacement présélectionné dans un arrangement d'oligonucléotides immobilisés.

4. Procédé pour détecter la présence ou l'absence d'un nucléotide spécifique d'allèle dans une séquence d'acides nucléiques cibles d'un échantillon d'acide nucléique, ledit procédé comprenant :
(a) la combinaison, dans les conditions d'une hybridation, pour former un duplex
(i) d'un échantillon d'acide nucléique qui peut comprendre une séquence d'acides nucléiques cibles,
(ii) une amorce spécifique d'allèle ayant une portion 3' et une portion 5', où ladite portion 3' est complémentaire d'une séquence cible immédiatement adjacente du nucléotide spécifique d'allèle, et où ladite portion 5' est complémentaire d'un acide nucléique présélectionné qui est différent de ladite séquence cible, et où ladite séquence d'acide nucléique présélectionnée est immobilisée sur un emplacement présélectionné d'un arrangement de séquences d'acides nucléiques immobilisées et présélectionnées et d'un support solide,
(b) l'addition, au produit de l'étape (i), d'une ADN-polymérase et de désoxynucléosides triphosphates, dont l'un est un nucléotide spécifique d'allèle contenant un marqueur, dans les conditions d'une hybridation, pour mettre à disposition un produit d'extension marqué de ladite amorce, qui comprend ladite séquence cible et ledit nucléotide spécifique d'allèle, s'il est présent ;
(c) le criblage du produit d'extension d'amorce marqué, spécifique d'allèle, de (b), par combinaison de la portion 5' avec l'arrangement de séquences d'acides nucléiques immobilisées et présélectionnées sur un support solide, le marqueur identifiant le nucléotide spécifique d'allèle, et l'emplacement de la séquence d'acides nucléiques présélectionnée immobilisée étant associé au locus de la séquence d'acides nucléiques cible de l'échantillon d'acides nucléiques.

5. Procédé selon la revendication 4, dans lequel la séquence d'acides nucléiques présélectionnée est un oligonucléotide immobilisé sur un emplacement présélectionné d'un arrangement d'oligonucléotides immobilisés sur un support solide.

6. Procédé selon la revendication 4, dans lequel ledit échantillon d'acides nucléiques peut contenir une pluralité de séquences d'acides nucléiques cibles, et dans lequel ledit échantillon d'acides nucléiques est soumis à une amplification par réaction en chaîne par polymérase avec au moins une paire d'amorces oligonucléotidiques pour au moins deux desdites séquences d'acides nucléiques cibles.

7. Procédé selon la revendication 6, dans lequel lesdites séquences d'acides nucléiques cibles sont des allèles l'une de l'autre, la portion 3' de ladite amorce est positionnée en un point immédiatement adjacent du nucléotide variant responsable de l'allélisme, et lesdites réactions d'extension d'amorce sont réalisées d'une manière indépendante avec chacun des quatre désoxynucléosides triphosphates, de façon que seulement une réaction d'extension d'amorce se produise pour chacun desdits allèles, lesdits désoxynucléosides triphosphates étant marqués de telle sorte qu'il y ait, pour chacun desdits allèles, production d'un produit d'extension d'amorce marqué unique.

8. Procédé selon la revendication 4, dans lequel la portion 5' est complémentaire d'une séquence d'acides nucléiques présélectionnée et générée au hasard.

9. Procédé selon la revendication 4 ou 8, dans lequel la longueur, la composition en bases et le pourcentage de chaque base dans chaque séquence d'acides nucléiques présélectionnée immobilisée sont essentiellement les mêmes, pour permettre à une hybridation entre ladite portion 5' et ladite séquence d'acides nucléique présélectionnée d'être mise en oeuvre à la même température pour toutes lesdites séquences d'acides nucléiques présélectionnées immobilisées.
